# EUROPEAN PATENT SPECIFICATION

(11) **EP 0 279 079 B1**
(45) Date of publication and mention of the grant of the patent: **09.04.1997**
(21) Application number: 87119377.7
(22) Date of filing: 30.12.1987
(51) Int. Cl.: A61K 31/52

(54) **Purine derivatives as interleukin-1 inhibitors**
Purinderivate als Interleukin-1-Inhibitoren
Dérivés de la purine comme inhibiteurs d'interleukine 1

(30) Priority: 31.12.1986 US 947905
(43) Date of publication of application: 24.08.1988
(73) Proprietor: HOECHST MARION ROUSSEL, Inc., Kansas City, Missouri 64137-1405 (US); THE UNIVERSITY OF VIRGINIA ALUMNI PATENTS FOUNDATION, Charlottesville, Virginia 22903 (US)
(72) Inventor: Mandell, Gerald L.,, Charlottesville, Virginia 22903 (US); Sullivan, Gail W.,, Charlottesville, Virginia 22903 (US); Novick, William J., Jr.,, Lebanon, New Jersey 08833 (US)
(74) Representative: Grünecker, Kinkeldey, Stockmair & Schwanhäusser Anwaltssozietät

(56) References cited:
- WO-A-85/02542
- JOURNAL OF MEDICINE, vol. 14, no. 2, 1983, pp. 137-145, PJD Publications Limited, Westbury, NY, US; J.C. ROBIN et al.: "Studies on osteoporoses. XI. Effects of a methylxanthine derivative"
- CHEMICAL ABSTRACTS, vol. 103, no. 17, October 28, 1985, p. 26, abstract no. 134498x, Columbus, Ohio, US; G.W. SULLIVAN et al.: "Enhancement of chemotaxis and protection of mice from infection"
- DTSCH. MED. WSCHR., vol. 111, no. 26, 1986, pp. 1032-1038, Georg Thieme Verlag Stuttgart, New York, US; G. RAMADORI et al.: "Interleukin-1: ein neues hormon"

## Description

### BACKGROUND OF THE INVENTION

### Field of the Invention:

This invention pertains to the inhibition of interleukin-1 in humans and mammals and specifically to inhibit the activity of interleukin-1 to arrest or alleviate certain disease and inflammation situations.

### Discussion of Background:

Interleukin-1 is a biological substance produced, in mammals by macrophages. The substance has been determined to effect a wide variety of cells and tissues, both in vitro and in vivo. Research has demonstrated interleukin-1 (IL-1) to be an important, and even critical, mediator in a wide variety of inflammatory states and diseases. The inhibition of IL-1, would be obviously of benefit in controlling, reducing and alleviating many of these conditions.

Detection of IL-1 activity, and its inhibition, can be relatively easily documented, through in vitro analysis of polymorphonuclear neutrophil behavior. Among other activities attributed to IL-1 is the promotion of leukocyte adherence and the inhibition of neutrophil chemotaxis, both directly contributing to disease and inflammation syndromes.

Yet, despite the obvious desirability of IL-1 activity and the ease with which inhibition can be detected, in vitro, there is, to date, no known inhibitor of IL-1 acceptable for in vivo administration.

### SUMMARY OF THE INVENTION

It is one object of this invention to meet the above-identified needs of the prior art.

It is another object of this invention to provide a method of inhibiting IL-1 activity.

It is yet a further object of this invention to identify a class of compounds which may be successfully employed in alleviating conditions caused by, or mediated by, IL-1. These and other objects made clear below are achieved by a class of compounds which includes pentoxifylline and related compounds, which show, even at low concentrations, marked inhibition of known IL-1 activity, as demonstrated through easily verified in vitro tests, noted above.

The IL-1 inhibitors of the claimed invention are of the general formula I 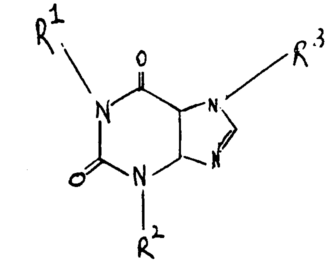 wherein at least one of R¹ and R³ is either (a) a branched hydroxyalkyl group of the formula 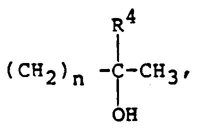 with a tertiary alcohol function, in which R⁴ stands for an alkyl group with 1 to 3 carbon atoms and n stands for a whole number from 2 to 5, the other R¹ or R³ group that may optionally be present stands for a hydrogen atom or an aliphatic hydrocarbon group R⁵ with up to 6 carbon atoms, whose carbon chain may be interrupted by up to 2 oxygen atoms or may be substituted with a hydroxy or oxo group, or (b) at least one of R¹ or R³ is an oxoalkyl group of the formula 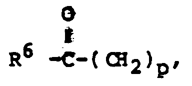 wherein R⁶ is C₁-C₆ alkyl, and p = 2, or 4. The other R¹ or R³ being defined as above; and R² represents an alkyl group with 1 to 4 carbon atoms.

Exemplary within the general formula, and established as an effective IL-1 inhibitor, is the well known and commercially available pharmaceutical pentoxifylline. Although this compound has been used, for some time, as a pharmaceutical (clinical trials in 1971) it has not been reported effective as a IL-1 inhibitor. It has been demonstrated in promoting directed migration of leukocytes. Other, related compounds, identified by their respective values for R₁ - R₃ are related below.

Because IL-1 has been implicated in such a wide variety of mammalian conditions, this invention has a similarly broad scope of application. Among the conditions that may be treated or alleviated by the inhibition of IL-1 are: sepsis, septic shock, endotoxic shock, gram negative sepsis, toxic shock syndrome, adult respiratory distress, cachexia secondary to infection or malignancy, cachexia secondary to AIDS, rhematory arthritis, gouty arthritis, and the inhibition of an immune response by inhibiting the activity of IL-1 or other leukocyte derived cytokines contained in LPS- or C. albicans-stimulated conditioned medium of mononuclear leukocytes. By reference to the specific cause of the disease condition, the more generic term "trauma" may be used. The term "trauma" refers broadly to cellular attack by foreign bodies and physical injury of cells. Included among foreign bodies are microorganisms, particulate matter, chemical agents, and the like. Included among physical injuries are, mechanical injuries such as abrasions, lacerations, contusions, wounds, and the like, thermal injuries such as those resulting from excessive heat or cold, electrical injuries such as those cause by contact with sources of electrical potential, and radiation damage caused, for example, by prolonged, extensive exposure to infrared, ultraviolet or ionizing radiations.

Microorganisms comprise bacilli, fungi and yeast, viruses parasites, and the like. Representative bacilli are:
a. Actinomyces spp.;
b. Bacteroides spp.;
c. Corynebacterium spp.;
d. Enterobacteriacea;
e. Enterococcus;
f. Haemophilus spp.;
g. Micrococcus spp.;
h. Neissera spp.;
i. Staphylococcus aureus;
j. Streptococcus pneumoniae;
l. Clostridium spp.;
m. Streptococcus agalactiae;
n. Bacillus spp.;
o. H. influenzae;
p. Moraxella spp.;
q. Mycobacteria spp.;
r. Pseutodomonas aeruginosa;
s. Vibrio spp.; and
t. Mycoplasma.

Representative fungi and yeast are:
a. Microspurum;
b. Blastomyces;
c. Histoplasma;
d. Aspergillus;
e. Cryptococcus;
f. Candida;
g. Coccidioides; and
h. Candida albicans.

Representative viruses are:
a. Rhinovirus;
b. Parainfluenza;
c. Enterovirus;
d. Influenza;
e. Chlamydiae;
f. Smallpox and vaccinia;
g. Herpes simplex;
h. Measles;
i. Rubella;
j. Arbovirus (Western, Eastern and Venezuelan equine encephalitis, and California encephalitis);
k. Rabies;
l. Colarado tick fever;
m. Yellow fever;
n. Dengue;
o. Virus B (HB Ag); and
p. Virus A (HAV).

Representative parasites are:
a. Trypanosoma cruzi;
b. Entamoeba histolytica;
c. Leishmania brasiliensis;
d. Leishmania tropica;
e. Leishmania donovani
f. Toxiplasma gondii;
g. Plasmodium falcipraum;
h. Trypanosoma rhodesiense;
i. Lia loa;
j. Trichomonas hominis;
k. Schistosoma japonicum;
l. Schistosoma mansoni; and
m. Fasciola hepatica.

Particulates include silica, asbestos, monosodium urate, cotton fibers, coal dust, beryllium, and the like.

Chemical agents include heavy metals such as lead, chromium, mercury, arsenic, and the like, organic solvents such as trichloroethylene, and the like, herbicides such as trichlorophenoxyacetic acid and the like, and pesticides such as mirex and the like. In addition, inhibition of IL-1 will enhance phagocyte activity in stored blood and blood products.

### DESCRIPTION OF THE PREFERRED EMBODIMENTS

Inhibition of IL-1 activity can be achieved by the administration of compounds of the formula I to the host or patient 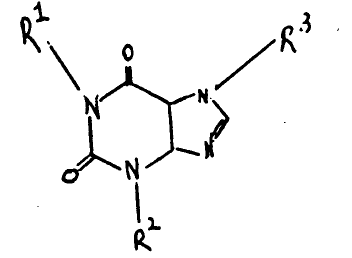 to be treated. As noted, among these compounds is the commercially available pentoxifylline. A host of other compounds within the general formula I have been identified as demonstrating IL-1 inhibiting activity. Among these compounds are those identified by their R substituents set forth below.

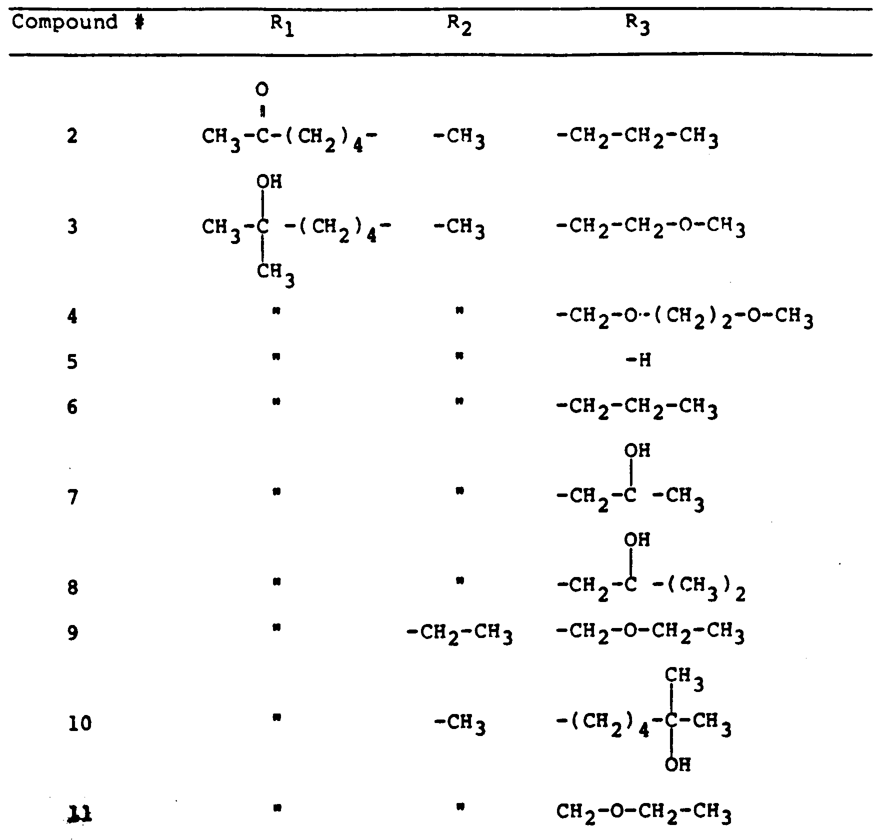

When introduced into polymorphonuclear neutrophil (PMN) incubations provided with IL-1, or incubated in lipopolysaccharide stimulated mononuclear leukocyte condition medium, the compounds of the claimed invention decreased PMN adherence, even at relatively low concentrations (0.1 of micrograms/ml).

Similarly, the presence of the compounds of the claimed invention promoted directed migration of PMN, which migration is inhibited by the presence of IL-1. The demonstrated inhibition of IL-1 by these compounds is, of course, suggestive of clinical effectiveness in the above-identified areas, and additional conditions. Appropriate dosages will vary with the condition and individual.

### Preparation of Compounds

As noted, among the compounds embraced in this invention is pentoxifylline (trental). Other compounds can be prepared according to the disclosure of U.S. Patent 3,737,433 and Belgium Patent 831,051 (where R¹/R³ are oxoallyl). For the cases where at least one of R¹/R³ is a tertiary alcohol reference may be had to the international application PCT-EP-86-00401, July 8, 1986 claiming German priority of July 8, 1985. This application addresses, as its invention, a variety of embodiments of synthesis routes for the xanthines embraced in the current invention.

An example of one embodiment consists of
a) reacting 3-alkylxanthines of Formula II 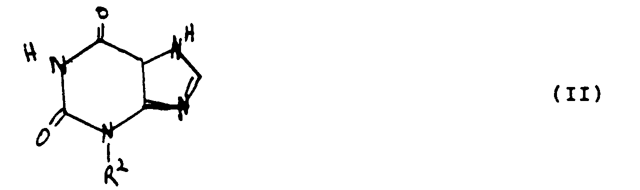 in which the R² represents alkyl with up to 4 carbon atoms, with alkylating agents of Formula III 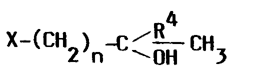 in which X
   stands for halogen, preferably chlorine, bromine, or iodine, or a sulfonic acid ester group or a phosphoric acid ester group and R⁴ and n have the meanings mentioned above, to obtain compounds of Formula Ib 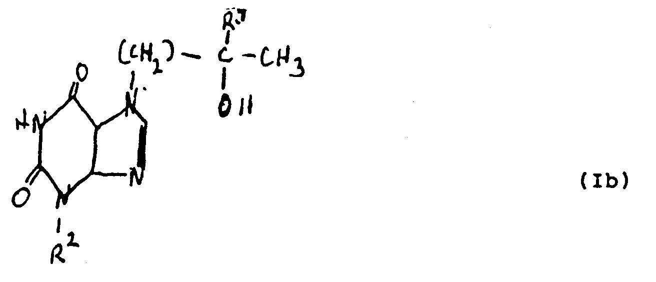 with a tertiary hydroxyalkyl group in the position of R³ and hydrogen in the position of R¹, and
a₁) alkylating this with the same or a different alkylating agent of Formula III to obtain compounds pursuant to the invention of Formula Ic 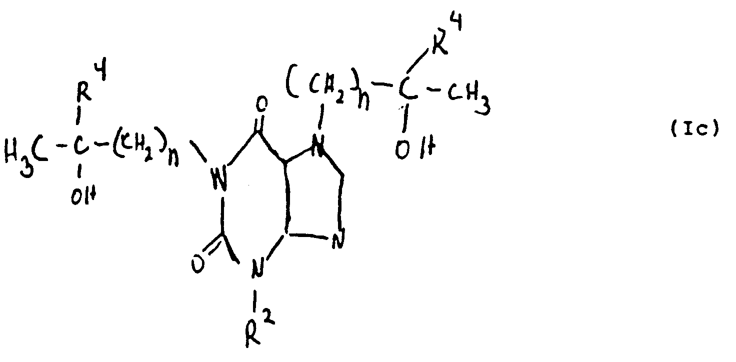 with two identical or different tertiary hydroxyalkyl groups in the positions of R¹ and R³, or
a₂) converting it with a compound of the Formula R⁵-X (IV), in which X has the meaning given in Formula III and R⁵ has the meaning indicated above, into compounds of Formula Id 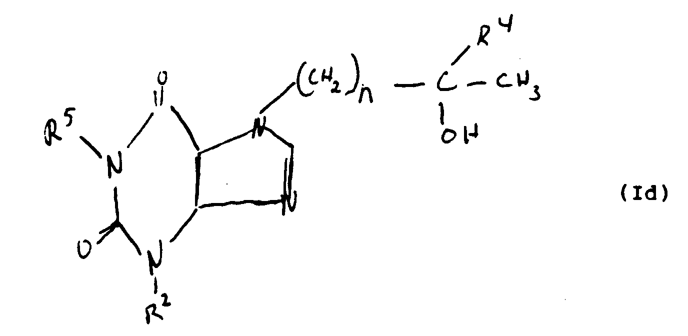 in all cases preferably operating in the presence of basic media or using the xanthines in the form of their salts.

Another form of embodiment b) consists of substituting 1,3-dialkylated xanthines of Formula V 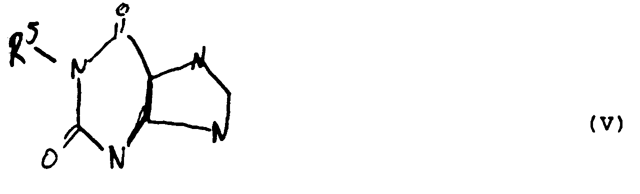 in the 7-position, preferably in the presence of basic media or in the form of their salts, by one-step reaction with a compound of Formula III, to obtain compounds of Formula Id.

Another form of embodiment c) consists of first reacting the 3-alkylxanthines of Formula II, likewise preferably in the presence of basic media or in the form of their salts, with a compound of the Formula R¹⁵-X (IVa) with the formation of 3,7-disubstituted xanthines of Formula VI 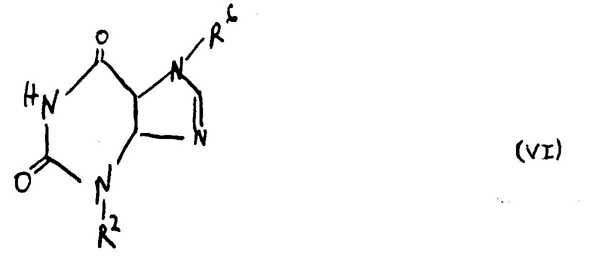 in which R¹⁵ has the meaning mentioned for R⁵ or stands for benzyl or diphenylmethyl, and then substituting them in the 1-position, again preferably in the presence of basic media or in the form of their salts, with a compound of Formula III, with compounds of Formula Ie 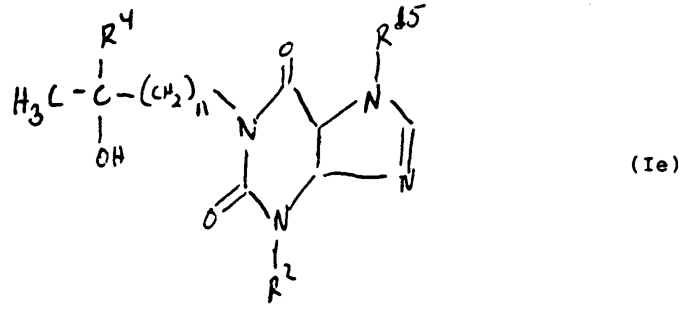 being obtained, and converting the compounds of Formula Ie in which R¹⁵ represents a benzyl or diphenylmethyl group or an alkoxymethyl or alkoxyalkoxymethyl group, under reducing or hydrolytic conditions, into compounds pursuant to the invention of Formula If 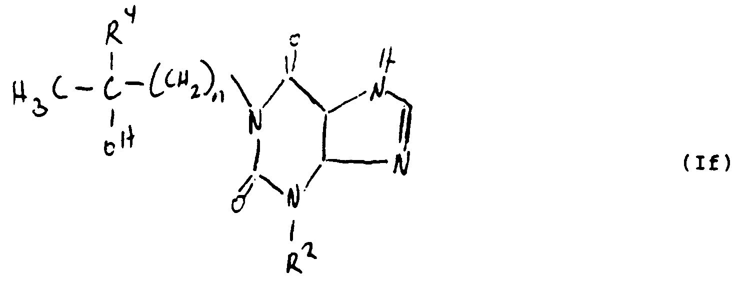 that are subsequently reacted again, if desired, with a compound of Formula III or IV to obtain compounds pursuant to the invention of Formula Ic or Ie.

Another form of embodiment d) consists of reducing compounds of Formula Id or Ie pursuant to the invention in which R⁵ or R¹⁵ stands for an oxoalkyl group, with conventional reducing agents for the keto group to obtain the corresponding hydroxyalkylated xanthines pursuant to the invention.

The 3-alkyl- or 1,3-dialkylxanthines of Formula II or V used here as starting materials and the "alkylating agents" of Formulas III, IV, and IVa are known for the most part or can be prepared readily by methods disclosed in the literature. Thus, the tertiary alcohols of Formula III, for example, can be obtained by organometallic synthesis by reacting the sterically unhindered haloketones of the formula Hal-(CH₂)ₙ-CO-CH₃ (VIIa), in a so-called synthetic reaction with reductive alkylation of the carbonyl group, with alkylmetal compounds R⁴-M, especially of magnesium, zinc, or lithium, for example in the form of the alkylmagnesium halides R⁴-MgHal (Grignard compounds) or of the alkyllithium compounds R⁴-Li under the usual conditions (for example, see Houben-Weyl, Vol. VI/1 a, Part 2 (1980), pp. 928-40, especially pp. 1021 ff. and 1104-1112). In the same way, a reaction of the haloketones with the formula Hal-(CH₂)ₙ-CO-R⁴ (VIIb) with methylmagnesium halides or methyllithium likewise leads to the target.

The hydroxyketones corresponding to the formulas VIIa and VIIb can also be converted smoothly into diols with the alkylmetal compounds in the usual way, either directly or with temporary masking of the hydroxy group, for example by acetal formation with 5,6-dihydro-4H-pyran (for example, see Houben-Weyl, Vol. VI/1 a, Part 2 (1980), pp. 1113-1124), from which compounds of Formula III are formed by selective esterification of the terminal primary hydroxyl groups with sulfonyl or phosphoric halides or anhydrides, advantageously in the presence of basic media.

Other possibilities for the synthesis of the tertiary alcohol derivatives of Formula III consist of the monometallation of ω-chloro-1-bromoalkanes to obtain ω-chloroalkylmetal compounds, (Houben-Weyl, Vol. XIII/2 a (1973), pp. 102 and 319) and their subsequent reaction with the ketones R⁴-CO-CH₃, with the extent of byproduct formation from the alkanolates formed as intermediates because of their tendency toward ring closure with the elimination of metal salt being minimized by appropriate temperature control, or of using ω-halo-1-alkanols as starting materials, which are metallated in the usual way, preferably in the form of the tetrahydropyranyl-(2) ether or after alkanolate formation on the hydroxy group (MO-(CH₂)ₙ-Hal) with any desired alkylmetal compound (for example, see Houben-Weyl, Vol. XIII/2 a (1973, p. 113), then reacting them with the ketones R⁴-CO-CH₃ to obtain the diols mentioned in the preceding paragraph (Houben-Weyl, Vol. VI/1 a, Part 2 (1980), p. 1029), and subsequently selectively esterifying the primary hydroxy group with suitable sulfonic or phosphoric acid derivatives.

A convenient access to compounds of Formula III in which R⁴ represents a methyl group is also available through the reaction of ω-haloalkanoic acid alkyl esters (Hal-(CH₂)ₙ-COO-alkyl) with two equivalents of a methylmetal compound, with the ester reacting through the ketone to produce the tertiary alcohol with the introduction of two methyl groups (Houben-Weyl, Vol. VI/1 a, Part 2 (1980), pp. 1171-1174). In the same way, ω-hydroxycarboxylic acid esters can be converted into diols with methylmetal compounds with or without protection of the hydroxy group, for example in the form of tetrahydropyranyl-(2) or methoxymethyl ether, or optionally in the form of the lactones as cyclic esters (for example, see Houben-Weyl, Vol. VI/1 a, Part 2 (1980), pp. 1174-1179), from which active alkylating agents of Formula III can in turn be obtained by selective esterification of the primary hydroxyl group with sulfonic or phosphoric halides or anhydrides.

Suitable compounds of Formula III that can be prepared by the methods described above are thus the [(ω-1)-hydroxy-(ω-1)-methyl]butyl, -pentyl, -hexyl, and -heptyl, the [(ω-2)-hydroxy-(ω-2)-methyl]pentyl, -hexyl, -heptyl, and -octyl, and the [(ω-3)-hydroxy-(ω-3)-methyl]hexyl, -heptyl, -octyl, and -nonyl chlorides, bromides, iodides, sulfonates, and phosphates.

Among the compounds of Formula R⁵-X (IV) or R¹⁵-X (IVa) suitable for the introduction of R⁵ into the 1-or 7-position and of R¹⁵ into the 7-position of the xanthine skeleton, the alkoxymethyl and alkoxyalkoxymethyl derivatives occupy a special position inasmuch as their halides can indeed be used successfully as reactants but toxicological problems can arise, at least in large-scale use. For this reason, the use of the corresponding sulfonates is preferred in this special case, which are readily available, for example, by reacting mixed anhydrides of aliphatic carboxylic acids and aliphatic or aromatic sulfonic acids (M. H. Karger et al., J. Org. Chem. 36 (1971), pp. 528-531) with formaldehyde dialkyl acetals or dialkoxyalkyl acetals in a smooth and nearly quantitative reaction (M. H. Karger et al., J. Amer. Chem. Soc. 91 (1969), pp.5663-5665): 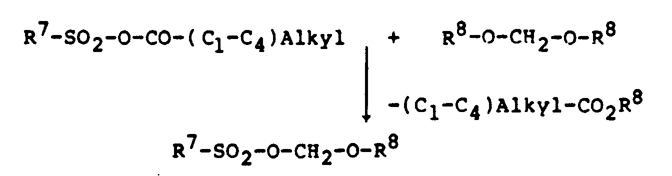 In this equation, R⁷ represents an aliphatic group such as methyl, ethyl, or trifluoromethyl, or an aromatic group, for example phenyl, 4-tolyl, or 4-bromophenyl, but preferably methyl or 4-tolyl, and R⁸ represents an alkyl or alkoxyalkyl group falling under the definition of R⁵ or R¹⁵.

The reaction can be carried out either in the substance or in an anhydrous aprotic solvent inert to the reactants at temperatures between -20° and +40°C, preferably between 0° and 20°C. No intermediate isolation of the highly reactive sulfonates, which are sensitive to hydrolysis and thermally labile, is necessary; they are preferably used immediately as crude products for the substitution on the nitrogen of the xanthines, with the usual addition of a basic condensing agent being unnecessary.

The reaction of the mono- or disubstituted xanthine derivatives Ib, If, II, V and VI with the alkylating agent involved of Formula III or IV or IVa is ordinarily done in a distributing agent or solvent inert to the reactants. Practical representatives are especially dipolar, aprotic solvents, for example formamide, dimethylformamide, dimethylacetamide, N-methylpyrrolidone, tetramethylurea, hexamethylphosphoric triamide, dimethyl sulfoxide, acetone, or butanone; however, alcohols such as methanol, ethylene glycol, and their mono- or dialkyl ethers with the alkyl group having 1 to 4 carbon atoms but both together having a maximum of 5 carbon atoms, ethanol, propanol, isopropanol, and the various butanols; hydrocarbons such as benzene, toluene, or xylenes; halogenated hydrocarbons such as dichloromethane or chloroform; pyridine, and mixtures of the solvents mentioned or their mixtures with water can also be used.

The "alkylation reactions" are suitably carried out in the presence of a basic condensing agent. Examples of materials suitable for this are alkali metal or alkaline earth hydroxides, carbonates, hydrides, alcoholates, and organic bases, such as trialkylamines (for example, triethyl- or tributylamine), quaternary ammonium or phosphonium hydroxides and crosslinked resins with fixed, optionally substituted ammonium or phosphomium groups. The xanthine derivatives can also be used in the alkylation reaction directly in the form of their separately prepared salts, such as the alkali metal, alkaline earth, or optionally substitued ammonium or phosphonium salts. The mono- and disubstituted xanthine derivatives can also be alkylated either in the presence of the aforementioned inorganic condensing agents or in the form of their alkali metal or alkaline earth salts with the assistance of so-called phase transfer catalysts, for example tertiary amines, quaternary ammonium or phosphonium salts, or crown ethers, preferably in a 2-phase system under the conditions of phase transfer catalysis. Among the suitable phase transfer catalysts that are generally commercially available are tetra(C₁-C₄)alkyl- and methyltrioctylammonium and -phosphonium salts, methyl-, myristyl-, phenyl-, and benzyltri(C₁-C₄)alkyl- and cetyltrimethylammonium as well as (C₁-C₁₂)alkyl- and benzyltriphenylphosphonium salts, with the compounds that have the larger and more symmetrically structured cation generally proving to be more effective.

The introduction of the groups Ia, R⁵, and R¹⁵ by the procedures described above is generally carried out at a reaction temperatures between 0°C and the boiling point of the particular reaction medium used, preferably between 20° and 130°C, optionally at elevated or reduced pressure, but ordinarily at atmospheric pressure, for which the reaction time can amount to less than 1 hour or up to several hours.

The reaction of the 3-alkylxanthines II to produce the compounds pursuant to the invention of Formula Ic requires the introduction of two tertiary hydroxyalkyl groups. either identical or different substituents can be linked to the xanthine skeleton in succession, or two identical hydroxyalkyl groups can be linked without isolation of intermediates in a single-pot reaction.

The reductive cleavage of the benzyl and diphenylmethyl group from compounds of Formula Ie with the formation of the xanthine atom in the 7-position, is carried out under standard conditions that were developed especially in the framework of the protective group technique in alkaloid and peptide syntheses and can thus be assumed to be widely known. Besides the chemical reduction, particularly of the benzyl compounds with sodium in liquid ammonia (Houben-Weyl, Vol. XI/1 (1957), pp. 974-975), the elimination of the two aforementioned aralkyl groups by catalytic hydrogenolysis, using a precious metal catalyst is also especially practical (Houben-Weyl, Vol. IX/1 (1957), pp. 968-971 and Vol. IV/1 c, Part 1 (1980), pp. 400-404). A lower alcohol is ordinarily used here as the reaction medium (optionally with the addition of formic acid or ammonia), or an aprotic solvent such as dimethylformamide or particularly glacial acetic acid, however, their mixtures with water can also be used. Especially suitable hydrogenation catalysts are palladium black and palladium on activated charcoal or barium sulfate, while other precious metals such as platinum, rhodium, and ruthenium frequently give rise to side reactions because of competitive ring hydrogenation and are therefore only conditionally usable. The hydrogenolysis is preferably carried out at temperatures between 20° and 100°C and at atmospheric pressure, or preferably slight excess pressure up to approximately 10 bar, with reaction times of a few minutes to several hours generally being needed.

The 1,3,7-trisubstituted xanthines of Formula Ie that have an alkoxymethyl or alkoxyalkoxymethyl group in the position of R¹⁵ represent O,N-acetals. Consequently, their substituents in the 7-position can be split off under the usual conditions of acid hydrolysis (cf. Houben-Weyl, Vol. VI/1 b (1984), pp. 741-745), with the 7H compounds of Formula I f likewise being formed. Examples of preferred groups that can be eliminated hydrolytically are the methoxy, ethoxy, and propoxymethyl groups as well as the methoxyethoxy- and ethoxyethoxymethyl groups. The reaction is advantageously carried out with heating in dilute mineral acids such as hydrochloric or sulfuric acid, optionally with the addition of glacial acetic acid, dioxane, tetrahydrofuran, or a lower alcohol as a solution promoter. Also useful are perchloric acid or organic acids such as trifloroacetic, formic, and acetic acid, in combination with catalytic amounts of mineral acids. The alkoxyalkoxymethyl compounds in particular can also be cleaved by using Lewis acids such as zinc bromide and titanium tetrachloride in anhydrous medium, preferably in dichloromethane or chloroform, with the 7-bromomethyl or 7-bromozinc derivatives formed as intermediates hydrolyzing spontaneously during the aqueous workup. In the cleavage in mineral acid solution, the reaction temperatures must be chosen so that no significant dehydration of the tertiary hydroxyalkyl group in the 1-position occurs; it should therefore be below 100°C as a rule.

The reduction of the xanthines of Formulas Id and Ie with an oxoalkyl group in the position of R⁵ or R¹⁵ to the corresponding hydroxyalkyl compounds can indeed take place in principle either with base metals or by catalytic hydrogenation, but the method of choice consists of the reaction occurring under very mild conditions and in high yields with simple metal hydrides (MHₙ), complex metal hydrides (M¹[M²Hₙ]ₘ), or organometallic hydrides (Houben-Weyl, Vol. IV/1 d (1981), pp. 267-282, and Vol. VI/1 b (1984), pp. 141-155). Of the numerous complex metal hydrides that can be used for the reduction of ketones, the most frequently used reagents might be mentioned, for example, lithium alanate, lithium borohydride, and especially sodium borohydride, that is easier to handle because of its lower reactivity and above all permits working in alcoholic, alcoholic aqueous, and pure aqueous solutions or suspensions. In addition to the otherwise customary inert solvents such as ethers (for example, diethyl ether, tetrahydrofuran, 1,2-dimethoxyethane), hydrocarbons and pyridine, nitriles such as acetonitrile can also be used as the reaction medium. The hydrogenation, which is suitably carried out at temperatures between 0°C and the boiling point of the particular solvent, but preferably at room temperature, generally occurs rapidly and is complete within several minutes to a few hours.

The tertiary hydroxyalkylxanthines of Formula I can also be prepared by reacting substituted xanthines of Formula VIII 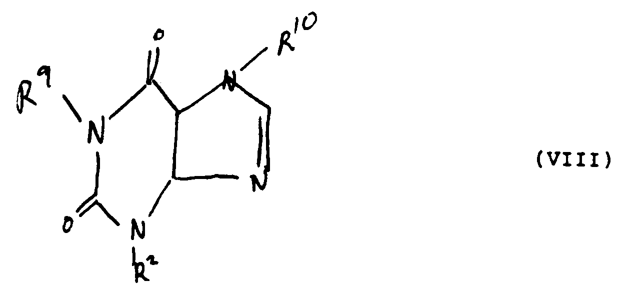
e) contains two identical or different groups of the formula -(CH₂)ₙ -CO-CH₃ (IXa) or (CH₂)ₙ-CO-R⁴ (IXb), or only one substituent of the Formula IXa or IXb, and hydrogen or the group R⁵ or R¹⁵ in the positions of R⁹ and R¹⁰, with (C₁-C₃)alkyl- or methylmetal compounds with reductive "alkylation" of the carbonyl groups to obtain the xanthines pursuant to the invention of Formulas Ib to If, or
f) metallating xanthines of Formula VIII that have two identical or different groups of the formula -(CH₂)ₙ-Hal (X), with Hal preferably standing for chlorine or bromine, or only one such group and hydrogen or the substituent R⁵ or R¹⁵ in the other position, in the terminal position, and then reacting them with the ketones of the formula R⁴-CO-CH₃ (XI) with reductive alkylation of the carbonyl group to obtain the xanthines of Formulas Ib to If pursuant to the invention, or
g) converting xanthines of Formula VIII with the group -(CH₂)ₙ-COO-(C₁-C₄)alkyl (XII) in the positions of R⁹ and/or R¹⁰ and optionally hydrogen or the group R⁵ or R¹⁵ in the other position, by means of two equivalents of a methylmetal compound per alkoxycarbonyl group, into xanthines of Formulas Ib to If in which R⁴ stands for methyl, or
h) converting xanthines of Formula VIII having two identical or different groups of the formula 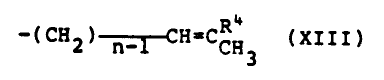 or only one such group and hydrogen or the group R⁵ or R¹⁵ in the position of R⁹ and R¹⁰, in which the group XIII can contain the C=C double bond also in position-isomeric arrangements on the branched carbon atom, for example, as -C=CH₂, by acid-catalyzed hydration obeying the Markownikoff Rule, into the xanthines of Formulas Ib to If pursuant to the invention, and if desired, then converting the tertiary hydroxyalkylxanthines of Formulas Ibʹ and If obtained pursuant to the invention by methods e) to h) that have a hydrogen atom in the 1- or 7-position, optionally in the presence of basic media or in the form of their salts, with the alkylating agents of Formula III or IV or IVa, into the trisubstituted compounds of Formulas Ic or Id or Ie, in which R², R⁴, R⁵, R¹⁵, and n in the formulas above have the meanings indicated above.

The 3-alkylated mono- or dioxoalkyl- (VIIIa), -(ω-haloalkyl)(VIIIb), -(ω-alkoxycarbonylalkyl)-(VIIIc), and -alkenylxanthines (VIIId) needed for this as starting materials are either known or can be prepared readily, for example, from the 3-alkylxanthines II and the sulfonyloxy- or haloketones VIIa and VIIb,ω-haloalkylsulfonates, or 1,ω-dihaloalkanes (cf., for example: V. B. Kalcheva et al., Journal fur prakt. Chemie 327 (1985) pp. 165-168), ω-sulfonyloxy-orω--halocarboxylic acid alkyl esters or sulfonyloxy-or haloalkenes corresponding to Formula XIII under the reaction conditions previously described in detail for the alkylation of mono- and disubstituted xanthines with the compounds of Formulas III and IV.

In the organometallic reactions of the xanthines VIIIa to VIIIc functionalized in the R⁹ and R¹⁰ groups, the procedure is the same in principle as described for the preparation of the tertiary alcohols of Formula III used as alkylating agents. Thus, the reductive alkylation of the ketones VIIIa and of the esters VIIIc can take place, for example, with alkylpotassium, -sodium, -lithium, -magnesium, -zinc, -cadmium, -aluminum, and -tin compounds. The recently recommended alkyltitanium and -zirconium compounds (D. Seebach et al., Angew. Chem. 95 (1983), pp. 12-26) can also be used. However, since the alkylmetal compounds of sodium and potassium have a tendency toward side reactions because of their high reactivity and those of zinc and cadmium are relatively sluggish, the alkyllithium and -magnesium (Grignard) compounds are ordinarily preferred.

The strongly nucleophilic organometallic compounds are very sensitive to hydrolysis and oxidation. Their safe handling therefore requires working in anhydrous medium, optionally under an inert gas atmosphere. The usual solvents or distributing agents are primarily those that are suitable also for the preparation of the alkylmetal compounds. Practical examples are especially ethers with one or more ether oxygen atoms, for example diethyl, dipropyl, dibutyl, or diisoamyl ether, 1,2-dimethoxyethane, tetrahydrofuran, dioxane, tetrahydropyran, furan, and anisole, and aliphatic or aromatic hydrocarbons such as petroleum ether, cyclohexane, benzene, toluene, xylenes, diethylbenzenes, and tetrahydronaphthalene; however, tertiary amines such as triethylamine, or dipolar aprotic solvents such as hexamethylphosphoric triamide, as well as mixtures of the solvents mentioned can also be used successfully. The reaction of the carbonyl compounds VIIIa and VIIIc with the Grignard compounds with the formula R⁴-MgHal can also beneficially be carried out by placing the organometallic compound in an ether and adding the ketone or the ester dropwise as a solution in dichloromethane or 1,2-dichloroethane. An addition of magnesium bromide is frequently recommended, which is able to increase the nucleophilicity of the organometallic compound because of its participation in the complex cyclic transition state.

The ketone or ester and the organometallic compound are generally combined at temperatures between -20° and 100°C, preferably between 0° and 60°, or at room temperature without external cooling, with the alkylmetal compound ordinarily being used in slight excess. The reaction is then ordinarily completed by brief heating under reflux, for which times of several minutes to a few hours are generally adequate. The alkanolate formed is preferably decomposed with aqueous ammonium chloride solution or dilute acetic acid.

Metallic magnesium and lithium are primarily suitable for the metallation of the ω-haloalkylxanthines VIIIb. On the other hand, the replacement of the halogen atom with lithium, which is also possible using organolithium reagents, generally 1-butyl-, 2-butyl, t-butyl-, or phenyllithium, plays a subordinate role. However, use is made especially of the Grignard compounds,advantageously preparing them in the ethers, hydrocarbons, tertiary amines, or aprotic solvents listed as particularly suitable for the reaction of the xanthines VIIIa and VIIIc with alkylmetal compounds, at temperatures between 25° and 125°C, preferably below 100°C. If the metallation reaction is carried out in hydrocarbons, then the addition of an ether such as tetrahydrofuran, or a tertiary amine such as triethylamine in stoichiometric amount frequently proves useful. The use of catalysts such as butanol, aluminum chloride, silicon tetrachloride, tetrachloromethane, and aluminum or magnesium alcoholates may also be helpful. In the halogen-metal exchange the chlorides ordinarily react more slowly than the corresponding bromides and iodides, but as a rule they provide better yields of organometallic compound. To accelerate the beginning of the reaction, the addition of some magnesium bromide, some grains of iodine, or several drops of bromine, tetrachloromethane, or methyl iodide with slight heating is frequently recommended. The Grignard compounds obtained are normally not isolated, but are reacted immediately with the ketones of Formula XI under the reaction conditions described for the reductive alkylation of the xanthines VIIIa and VIIIc.

The addition of water to the C=C double bond of the alkenylxanthines VIIId with the structural element of Formula XIII, in which the hydroxy group adds to the carbon atoms with the fewer hydrogens to form tertiary alcohols according to the Markownikoff Rule, ordinarily occurs in aqueous solution or suspension in the presence of strong acids such as sulfuric, nitric, or phosphoric acid. Hydrogen halides and sulfonic acids such as trifluoromethanesulfonic acid, acid exchange resins, boron trifluoride complexes, or oxalic acid can also be used as catalysts. However, it is preferred to operate in sulfuric acid, with an acid concentration of 50 to 65% and temperatures of 0° to 10°C being sufficient as a rule. However, lower or higher acid concentrations and/or reaction temperatures can sometimes also be used. In any case, the reaction temperature should be kept as low as possible since the reverse dehydration to the olefin can be disturbingly significant above approximately 60°C.

The addition of a solvent inert to acids such as 1,4-dioxane, benzene, or toluene sometimes also provides benefits. Since esters can form as intermediates in the acid-catalyzed hydration, particularly when using high acid concentrations, it is recommended to treat the reaction batch with a large amount of water with brief heating after the action of the acid for the purpose of ester hydrolysis, or to process the mixture in the alkaline range.

The experimental conditions for the optional conversion of the 1- and 7H-compounds Ib or If pursuant to the invention into the trisubstituted xanthines of Formulas Ic or Id or Ie by N-alkylation with the compounds III or IV or IVa have already been described above in detail.

Depending on the chain length of the alkyl group R⁴ (at least C₂) and/or the structure of the substituent R⁵ (for example, 2-hydroxypropyl), the tertiary hydroxyalkylxanthines of Formula I can have one or two asymmetric carbon atoms and can thus be present in stereoisomeric forms. This invention therefore concerns both the pure stereoisomeric compounds and their mixtures.

### Examples of Inhibition

To demonstrate the effectiveness of the claimed invention, compounds of the general formula I were tested to demonstrate inhibition of the activity of both in vitro-generated human IL-1 and purified human IL-1. Though a variety of compounds within the general formula I have been demonstrated to effectively inhibit the activities of IL-1, including the preferred compounds set forth above, they will be exemplified, below, with regard to the performance of pentoxifylline as a particularly preferred form of the invention.

Purified human IL-1 was obtained from Cistron Technology, Pinebrook, New Jersey. As is known, the production of IL-1 from macrophages or circulating monocytes can be stimulated by the presence of bacterial lipopolysaccharides. Stites et al, Basic and Clinical Immunology, page 87 (1984). Accordingly, in vitro-generated IL-1 was obtained through the incubation of mononuclear leukocytes. Mononuclear leukocytes (3 x 10⁶/ml) from ficoll-hypaque separation were incubated in medium 199 (M199) containing 10% fresh autologous serum with or without lipopolysaccharides 5ng/ml or with or without supernatant from C. albicans culture for 18 hours at 37° C (10% CO₂) in LAB-TEK Flaskettes (Miles Inc., Naperville, Illinois). The suspension was centrifuged (150g X 10 minutes) and the supernatant filtered (0.45 micron 4) and frozen (-70° C).

As reported below, not only is the adherence of polymorphonuclear neutrophil (PMN) caused by IL-1 inhibited by the compounds of general formula I, but the inhibition of normal chemotaxis of PMN caused by IL-1 was also reduced by the presence of the compounds of general formula I. PMN chemotaxis was assayed under agarose by the method of Nelson, Quie and Simmons. Neutrophils were placed in the center well of a triplet and the chemoattractant (FMLP 10⁻⁷M) was placed in one outer well and M199 was placed in the opposite well. Following 2 hours incubation at 37° C the plates were fixed and stained and the zones of migration measured. The directed migration was the distance in mm that the leading front of PMN moved towards the chemoattractant.

Quantitative demonstration of the inhibition of the effects of IL-1 on a) the adherence of PMN and b) chemotaxis of PMN is set forth below.

### A. The effect of LPS stimulated mononuclear leukocyte conditioned medium on PMN adherence: How pentoxifylline modulates this effect

Purified PMN (5 x 10⁶/ml) were incubated 30 minutes at 37°C in M199, "NONE", LPS (1ng/ml), "LPS", mononuclear leukocyte conditioned medium, "CONT KINES", or in LPS stimulated mononuclear leukocyte conditioned medium, "LPS KINES". One ml was pipetted onto the top of a nylon fiber column and incubated at 37°C for 30 minutes. The PMN in the effluent samples were counted and the percent adherence of PMN on the column determined.

Pentoxifylline (50 miccrograms/ml) decreased PMN adherence under all four experimental conditions.

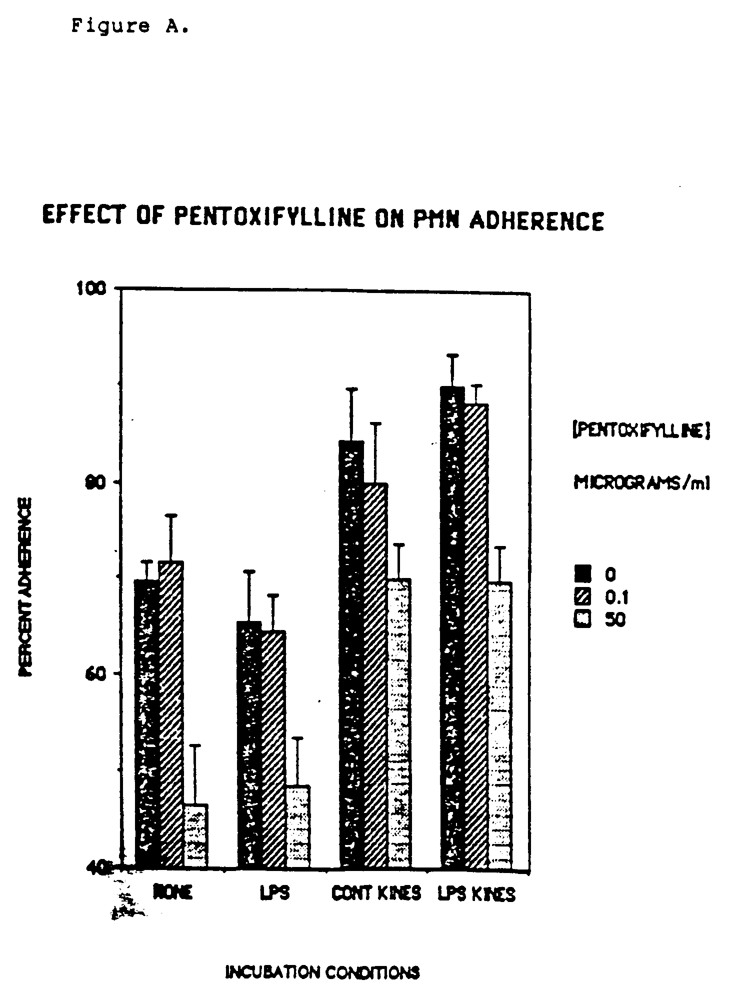

### B. The effect of IL-1 and LPS stimulated mononuclear leukocyte conditioned medium on PMN adherence: How pentoxifylline modulates this effect

Two tenths ml of purified PMN (1 x 10⁷/ml) was incubated 30 minutes at 37°C in M199, "control", or M199 containing IL-1 (80U/ml), "IL-1", or in LPS stimulated mononuclear leukocyte conditioned medium, "LPS KINE", with or without pentoxifylline (0.1 or 50 micrograms/ml).

Following incubation the samples were diluted to a final concentration of 5 x 10⁶/ml with M199 (2% serum). One ml was placed onto the top of a nylon fiber column and incubated at 37°C for 30 minutes. The PMN in the effluent samples were counted and the percent adherence of PMN on the nylon column calculated.

Both pentoxifylline 0.1 and 50 micrograms/ml decreased PMN nylon adherence under the three experimental conditions.

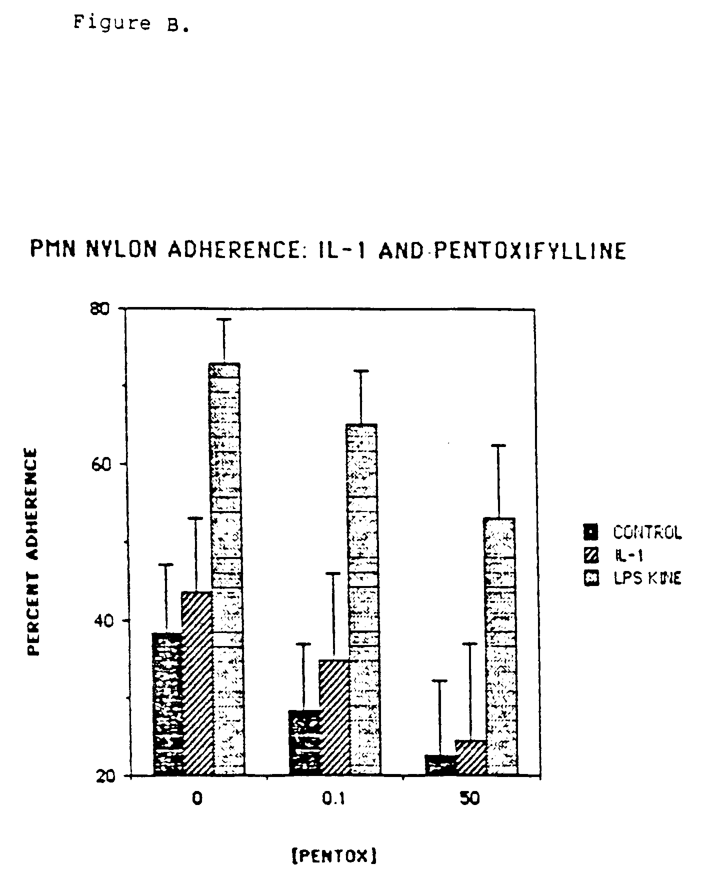

### C. Effect of LPS stimulated mononuclear leukocyte conditioned medium on PMN directed migration: Modulation of this effect by pentoxifylline

Pure PMN (5 x 10⁶/ml) were incubated for 30 minutes at 37°C with or without pentoxifylline (0.1 or 50 micrograms/ml) in M199 2% serum, "NO ADD", M199 2% serum containing LPS (1ng/ml), "LPS", mononuclear leukocyte conditioned M199 2% serum, "CONT KINE", or LPS stimulated mononuclear leukocyte conditioned M199 2% serum, "LPS KINE". The PMN were concentrated 10 fold prior to application in the under agarose chemotaxis assay.

Pentoxifylline (50 and 0.1 micrograms/ml) increased directed migration inhibited by "LPS KINE".

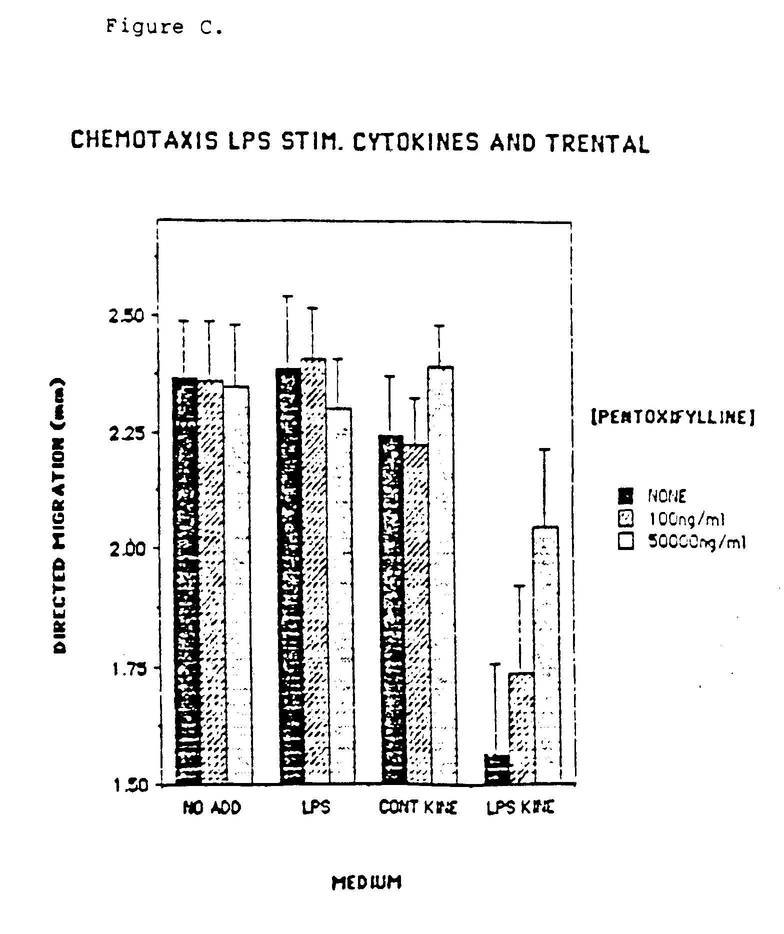

### D. Effect of C. albicans stimulated mononuclear leukocyte conditioned medium on PMN directed migration: Modulation of this effect by pentoxifylline

Pure PMN (5 x 10⁶/ml) were incubated for 30 minutes at 37°C with or without pentoxifylline (0.1 or 50 micrograms/ml) in M199 5% serum, "NO ADD", M199 5% serum containing supernatant from C. albicans culture, "C.ALB", mononuclear leukocyte conditioned M199 5% serum, "CONT KINE", or C. albicans stimulated mononuclear leukocyte conditioned M199 5% serum, "C. ALB KINE". The PMN were concentrated 10 fold prior to application in the under agarose chemotaxis assay.

Pentoxifylline (50 micrograms/ml) increased directed migration inhibited by "C.ALB KINE" and "CONT KINE".

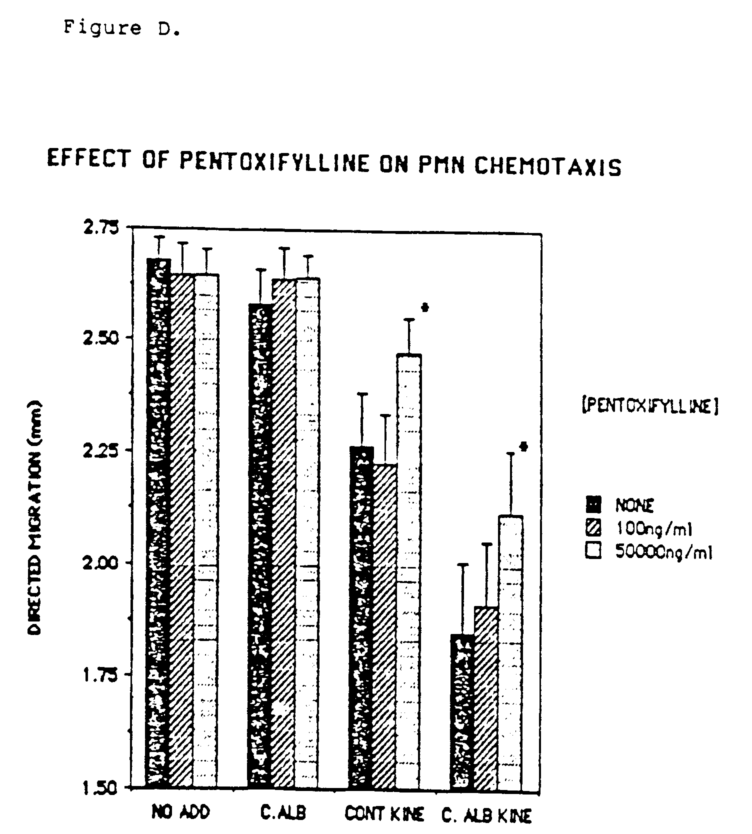

### E. Effect of interleukin-1 on PMN directed migration: Modulation of this effect by pentoxifylline

Pure PMN (5 x 10⁶/ml) were incubated for 30 minutes at 37°C with or without pentoxifylline (0.1 or 50 micrograms/ml) in minimum essential medium (MEM) or MEM containing IL-1 at 0 to 80 Units/ml. The PMN were concentrated 10 fold prior to application inthe under agarose chemotaxis assay.

Pentoxifylline (0.1 or 50 micrograms/ml) increased directed migration inhibited by interleukin-1.

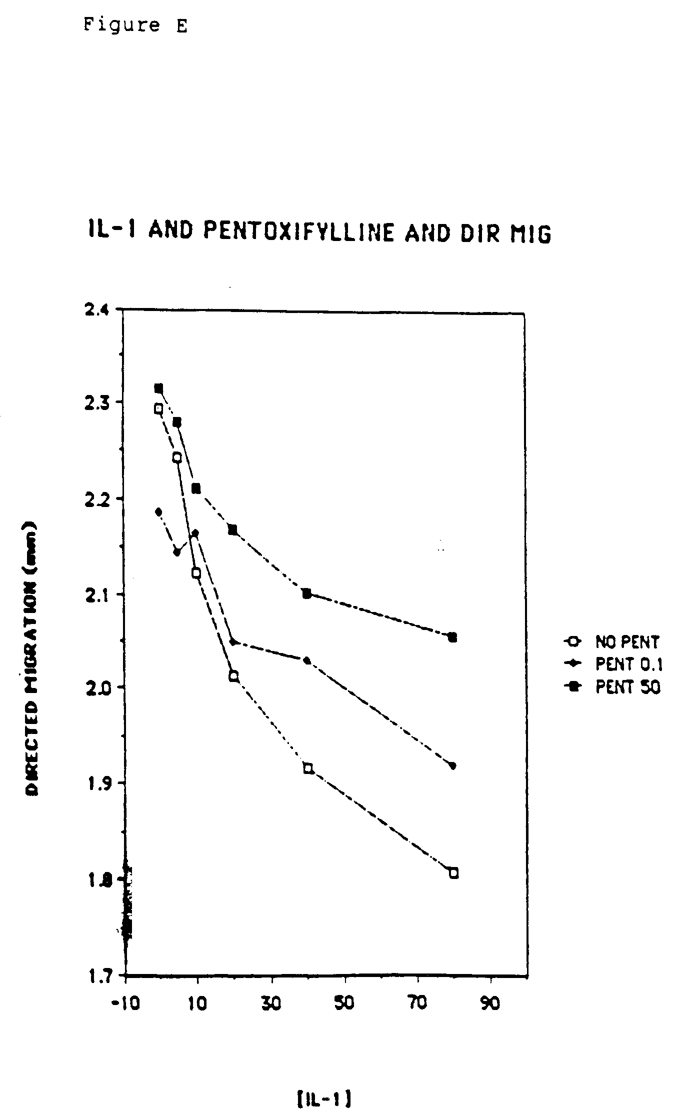

While dosage values will vary with the specific disease condition to be alleviated, good results are achieved when the xanthines of Formula I are administered to a subject requiring such treatment as an effective oral, parenteral or intravenous dose or from 0.10 to 25.0 mg/kg of body weight per day. A particularly preferred effective amount is about 1.0 mg/kg of body weight per day. In general, daily dosages will vary from 10-1000 mg, preferably 100-600 mg per day. It is to be understood, however, that for any particular subject, specific dosage regimens should be adjusted to the individual need and the prefessional judgement of the person administering or supervising the administration of the aforesaid compound. It is to be further understood that the dosages set forth herein are exemplary only and they do not, to any extent, limit the scope or practice of the invention.

Effective amounts of the xanthines may be administered to a subject by any one of various methods, for example, orally as in capsule or tablets, or parenterally in the form of sterile solutions. The xanthines, while effective themselves, may be formulated and administered in the form of their pharmaceutically acceptable addition salts for purposes of stability, convenience of crystallization, increased solubility and the like.

Preferred pharmaceutically acceptable addition salts include salts of mineral acids, for example, hydrochloric acid, sulfuric acid, nitric acid and the like, salts of monobasic carboxylic acids such as, for example, acetic acid, propionic acid and the like, salts of dibasic carboxylic acids such as, for example, maleic acid, fumaric acid, oxalic acid and the like, and salts of tribasic carboxylic acids such as, for example, carboxysuccinic acid, citric acid and the like.

The xanthines may be administered orally, for example, with an inert diluent or with an edible carrier. They may be enclosed in gelatin capsules or compressed into tablets. For the purpose of oral therapeutic administration, the aforesaid compounds may be incorporated with excipients and used in the form of tablets, troches, capsules, elixirs, suspensions, syrups, wafers, chewing gums and the like. These preparations should contain at least 0.5% of active compound, but may be varied depending upon the particular form. The amount of xanthine in such a compositions is such that a suitable dosage will be obtained. Preferred compositions and preparations according to the present invention are prepared so that an oral dosage unit form contain between 1.0-300 mgs of active compound.

The tablets, pills, capsules, troches and the like may contain the following ingredients: a binder such as microcrystalline cellulose, gum tragacanth or gelatin; and excipient such as starch or lactose, a disintegrating agent such as alginic acid, Primogel, corn starch and the like; a lubricant such as magnesium stearate or Sterotes; a glidant such as colloidal silicon dioxide; and a sweetening agent such as sucrose or saccharin or flavoring agent such as peppermint, methyl salicylate, or orange flavoring may be added. When the dosage unit form is a capsule, it may contain, in addition to material of the above type, a liquid carrier such as a fatty oil. Other dosage unit forms may contain other various materials which modify the physical form of the dosage unit, for example, as coatings. Thus tablets or pills may be coated with sugar, shellac, or other enteric coating agents. A syrup may contain, in addition to the active compounds, sucrose as a sweetening agent and certain preservatives, dyes and colorings and flavors. Materials used in preparing these various compositions should be pharmaceutically pure and non-toxic in the amounts used.

For the purposes of parenteral therapeutic administration, the xanthines may be incorporated into a solution or suspension. These preparations should contain at least 0.1% of the aforesaid compound, but may be varied between 0.5% and about 50% of the weight thereof. The amount of active compound in such compositions is such that a suitable dosage will be obtained. Preferred compositions and preparations according to the present invention are prepared so that a parenteral dosage unit contains betwen 0.5 to 100 mgs of the active compound.

The solutions or suspensions may also include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates citrates or phosphates and agents for the adjustment of tonicity such as sodium chloride or dextrose. The parenteral preparation can be enclosed in ampules, disposable syringes or multiple dose vials made of glass or plastic.

Obviously, numerous modifications and variations of the present invention are possible in light of the above teachings. It is therefore to be understood that within the scope of the appended claims, the invention may be practiced otherwise than as specifically described herein.

## Claims (Claims for the following Contracting State(s): AT, BE, CH, DE, FR, GB, GR, IT, LI, LU, NL, SE)

1. Use of at least one compound of the general formula (I) 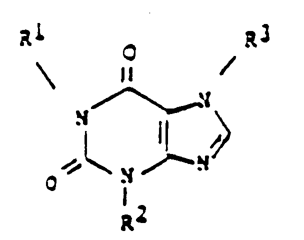 wherein at least one of R₁ and R₃ is either
a) a branched hydroxyalkyl group of the formula 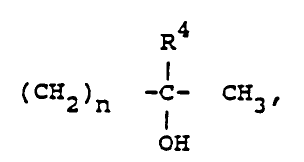 in which R⁴ stands for an alkyl group with 1 to 3 carbon atoms and n stands for a whole number from 2 to 5, the other R¹ or R³ group that may optionally be present stands for a hydrogen atom or an aliphatic hydrocarbon group R⁵ with up to 6 carbon atoms, whose carbon chain may be interrupted by up to 2 oxygen atoms or may be substituted with a hydroxy or oxo group, or
b) an oxoalkyl group of the formula 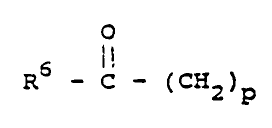 wherein R⁶ is C₁-C₆ and p is 2, or 4, the remaining R¹ or R³ being as defined above, and R² is an alkyl group C₁-C₄;
for the manufacture of a medicament for a mammal for
a) alleviating sepsis, septic shock, endotoxic shock, gram negative sepsis, toxic shock syndrome or adult respiratory distress; or
b) alleviating cachexia secondary to infection, malignancy or AIDS; or
c) inhibiting immune response by inhibiting the activity of IL-1 or other leukocyte derived cytokines contained in LPS- or C. albicans-stimulated conditioned medium of mononuclear leukocytes on polymorphonuclear neutrophils.

2. The use of claim 1 wherein said mammal is a human.

3. The use of claim 1 wherein said compound is pentoxiflylline.

4. The use of claim 1 wherein R¹ is a branched hydroxyalkyl group of the formula 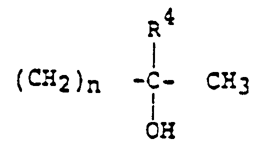 in which R⁴ stands for an alkyl group with 1 to 3 carbon atoms and n stands for a whole number from 2 to 5.

5. The use of claim 4 wherein:
R₁ is 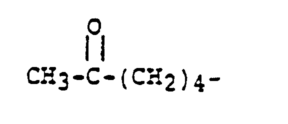
R₂ is
-CH₃
and
R₃ is
-CH₂-CH₂-CH₃.

6. The use of claim 4, wherein:
R₁ is 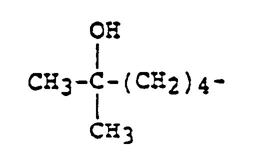
R₂ is
-CH₃
and
R₃ is
-CH₂-CH₂-O-CH₃.

7. The use of claim 4, wherein:
R₁ is 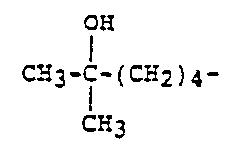
R₂ is
-CH₃
and
R₃ is
-CH₂-O-(CH₂)₂-O-CH₃

8. The use of claim 4, wherein:
R₁ is 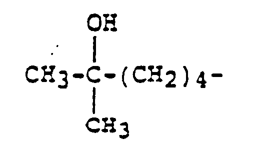
R₂ is
-CH₃
and
R₃ is
-H.

9. The use of claim 4, wherein:
R₁ is 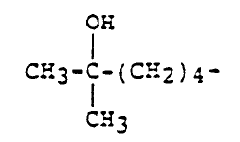
R₂ is
-CH₃
and
R₃ is CH₂-CH₂-CH₃

10. The use of claim 4, wherein:
R₁ is 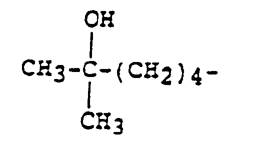
R₂ is
-CH₃
and
R₃ is

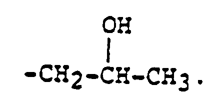

11. The use of claim 4, wherein:
R₁ is 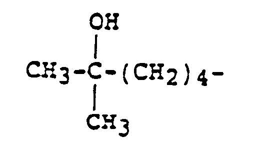
R₂ is
-CH₃
and
R₃ is

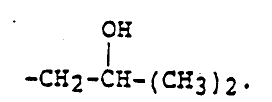

12. The use of claim 4, wherein:
R₁ is 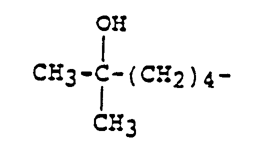
R₂ is
-CH₂-CH₃
and
R₃ is
-CH₂-O-CH₂-CH₃.

13. The use of claim 4, wherein:
R₁ is 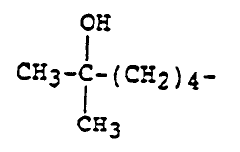
R₂ is
-CH₃
and
R₃ is

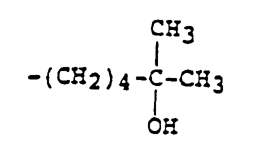

14. The use of claim 4 wherein:
R₁ is 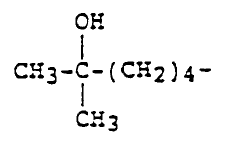
R₂ is
-CH₃
and
R₃ is
-CH₂-O-CH₂-CH₃.

15. Use of pentoxifylline for the manufacture of a medicament for inhibiting the activity of IL-1 or other leukocyte derived cytokines contained in LPS- or C. albicans-stimulated conditioned medium of mononuclear leukocytes in a human, except for treating osteoporosis.

16. Use according to claim 15 for the manufacture of a medicament for treating an adverse condition in a mammal caused by a virus.

17. The use of claim 16, wherein said mammal is a human.

## Claims (Claims for the following Contracting State(s): ES)

1. A process for the manufacture of a medicament for a mammal for
a) alleviating sepsis, septic shock, endotoxic shock, gram negative sepsis, toxic shock syndrome or adult respiratory distress; or
b) alleviating cachexia secondary to infection, malignancy or AIDS; or
c) inhibiting immune response by inhibiting the activity of IL-1 or other leukocyte derived cytokines contained in LPS- or C.albicans-stimulated conditioned medium of mononuclear leukocytes on polymorphonuclear neutrophils comprising the step of combining at least one compound of the general formula (I) 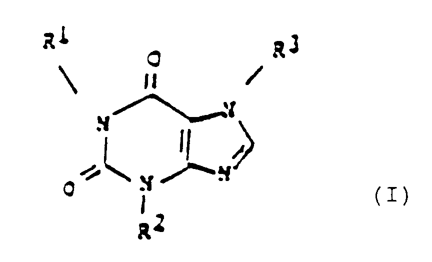
wherein at least one of R₁ and R₃ is either
a) a branched hydroxyalkyl group of the formula 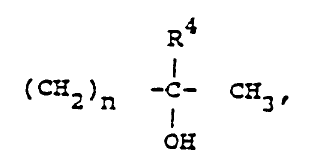 in which R⁴ stands for an alkyl group with 1 to 3 carbon atoms and n stands for a whole number from 2 to 5, the other R¹ or R³ group that may optionally be present stands for a hydrogen atom or an aliphatic hydrocarbon group R⁵ with up to 6 carbon atoms, whose carbon chain may be interrupted by up to 2 oxygen atoms or may be substituted with a hydroxy or oxo group, or
b) an oxoalkyl group of the formula 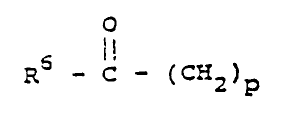 wherein R⁶ is C₁-C₆ and p is 2, or 4, the remaining R¹ or R³ being as defined above, and R² is an alkyl group C₁-C₄,
with diluents, carriers and/or excipients in a manner known per se.

2. The process according to claim 1, wherein said mammal is a human.

3. The process according to claim 1, wherein said compound is pentoxifylline.

4. The process according to claim 1 wherein R¹ is a branched hydroxyalkyl group of the formula 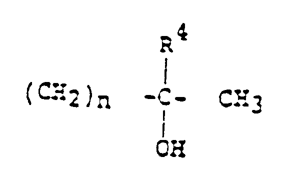 in which R⁴ stands for an alkyl group with 1 to 3 carbon atoms and n stands for a whole number from 2 to 5.

5. The process according to claim 4, wherein
R₁ is 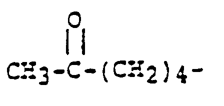
R₂ is
-CH₃
and
R₃ is
-CH₂-CH₂-CH₃.

6. The process of claim 4, wherein
R₁ is 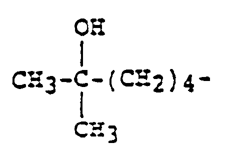
R₂ is
-CH₃
and
R₃ is
-CH₂-CH₂-O-CH₃.

7. The process of claim 4, wherein
R₁ is 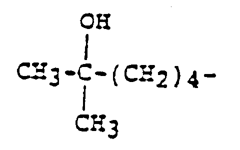
R₂ is
-CH₃
and
R₃ is
-CH₂-O-(CH₂)₂-O-CH₃

8. The process of claim 4, wherein
R₁ is 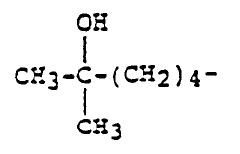
R₂ is
-CH₃
and
R₃ is
-H.

9. The process of claim 4, wherein
R₁ is 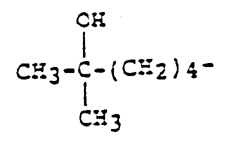
R₂ is
-CH₃
and
R₃ is CH₂-CH₂-CH₃

10. The process of claim 4, wherein
R₁ is 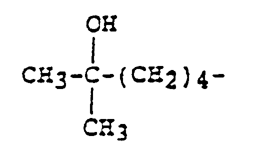
R₂ is
-CH₃
and
R₃ is

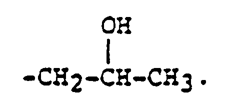

11. The process of claim 4, wherein
R₁ is 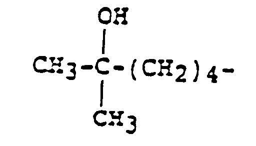
R₂ is
-CH₃
and
R₃ is

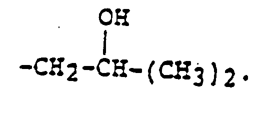

12. The process of claim 4, wherein
R₁ is 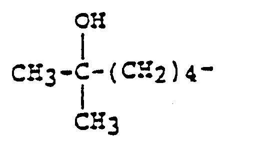
R₂ is
-CH₂-CH₃
and
R₃ is
-CH₂-O-CH₂-CH₃.

13. The process of claim 4, wherein
R₁ is 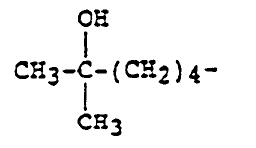
R₂ is
-CH₃
and
R₃ is

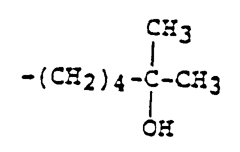

14. The process of claim 4, wherein
R₁ is 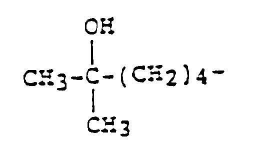
R₂ is
-CH₃
and
R₃ is
-CH₂-O-CH₂-CH₃.

15. A process for the manufacture of a medicament for inhibiting the activity of IL-1 or other leukocyte derived cytokines contained in LPS- or C.albicans-stimulated conditioned medium of mononuclear leukocytes in a human, except for treating osteoporosis, comprising the steps of combining pentoxifylline with diluents, carriers and/or excipients in a manner known per se.

16. A process according to claim 15, wherein a medicament for treating an adverse condition in a mammal caused by a virus is manufactured.

17. The process of claim 16, wherein said mammal is a human.

## Patentansprüche (Patentansprüche für folgende(n) Vertragsstaat(en): AT, BE, CH, DE, FR, GB, GR, IT, LI, LU, NL, SE)

1. Verwendung von mindestens einer Verbindung der allgemeinen Formel (I), 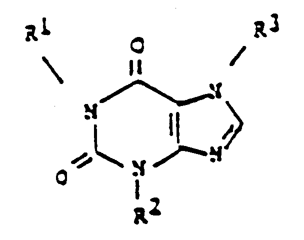 wobei mindestens einer der Reste R₁ und R₃ entweder
**a)** eine verzweigte Hydroxyalkylgruppe der Formel 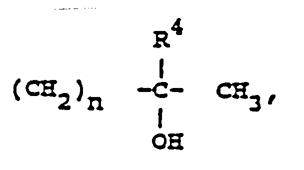 ist, in der R⁴ für eine Alkylgruppe mit 1 bis 3 Kohlenstoffatomen und n für eine ganze Zahl von 2 bis 5 steht, wobei die andere R¹ oder R³-Gruppe, die optional vorhanden sein kann, für ein Wasserstoffatom oder eine aliphatische Kohlenwasserstoffgruppe R⁵ mit bis zu 6 Kohlenstoffatomen steht, deren Kohlenstoffkette durch bis zu 2 Sauerstoffatome unterbrochen sein kann oder mit einer Hydroxy- oder Oxogruppe substituiert sein kann, oder
**b)** eine Oxoalkylgruppe der Formel 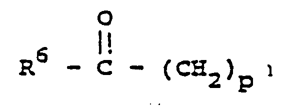 bedeutet, wobei R⁶ C₁-C₆ und p 2 oder 4 bedeutet, wobei die restlichen R¹ oder R³ wie oben definiert sind und R² eine C₁-C₄ Alkylgruppe ist.
für die Herstellung eines Medikaments für ein Säugetier, um
**a)** Sepsis, septischen Schock, endotoxischen Schock, gramnegative Sepsis, das toxische Schocksyndrom oder Atemschwierigkeiten bei Erwachsenen zu lindern; oder
**b)** Cachexie, infolge von Infektion, maligner Tumoren oder AIDS zu lindern; oder
**c)** die Immunantwort zu inhibieren, indem die Aktivität von IL-1 oder anderen von Leukozyten abstammenden Cytokinen, die in mit LPS- oder Candida albicans stimuliertem konditioniertem Medium mononucleärer Leucozyten enthalten sind, auf polymorphonucleäre neutrophile Granulozyten gehemmt wird.

2. Verwendung gemäß Anspruch 1, wobei das Säugetier ein Mensch ist.

3. Verwendung gemäß Anspruch 1, worin die Verbindung Pentoxifyllin ist.

4. Verwendung gemäß Anspruch 1, worin R¹ eine verzweigte Hydroxyalkylgruppe der Formel 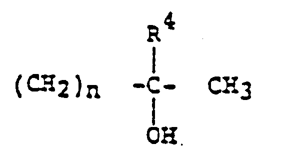 bedeutet, in der R⁴ für eine Alkylgruppe mit 1 bis 3 Kohlenstoffatomen und n für eine ganze Zahl von 2 bis 5 steht.

5. Verwendung gemäß Anspruch 4, worin
R₁ 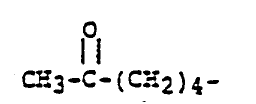 bedeutet.
R₂ -CH₃ bedeutet
und
R₃ -CH₂-CH₂-CH₃ bedeutet.

6. Verwendung gemäß Anspruch 4, worin
R₁ 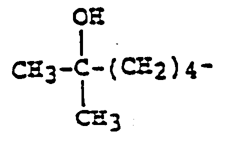
R₂ -CH₃ bedeutet
und
R₃ -CH₂-CH₂-O-CH₃ bedeutet.

7. Verwendung gemäß Anspruch 4, worin
R₁ 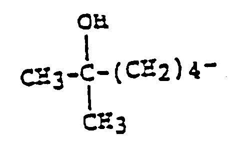 bedeutet,
R₂ -CH₃ bedeutet
und
R₃ -CH₂-O-(CH₂)₂-OCH₃ bedeutet.

8. Verwendung gemäß Anspruch 4, worin
R₁ 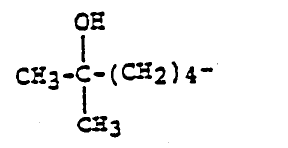 bedeutet,
R₂ -CH₃ bedeutet
und R₃ H bedeutet.

9. Verwendung gemäß Anspruch 4, worin
R₁ 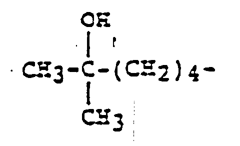
R₂ -CH₃ bedeutet
und R₃ -CH₂-CH₂-CH₃ bedeutet.

10. Verwendung gemäß Anspruch 4, worin
R₁ 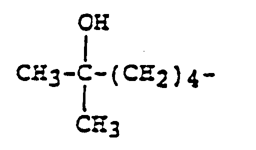 bedeutet
R₂ -CH₃ bedeutet
und
R₃ 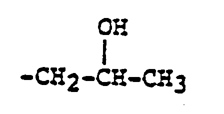 bedeutet.

11. Verwendung gemäß Anspruch 4, worin
R₁ 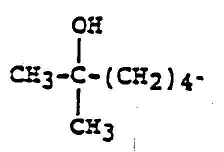 bedeutet,
R₂ -CH₃ bedeutet
und
R₃ 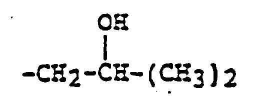 bedeutet.

12. Verwendung gemäß Anspruch 4, worin
R₁ 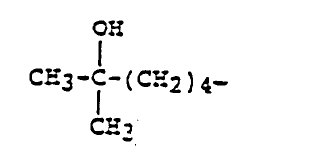 bedeutet,
R₂ -CH₂-CH₃ bedeutet
und
R₃ -CH₂-O-CH₂-CH₃ bedeutet.

13. Verwendung gemäß Anspruch 4, worin
R₁ 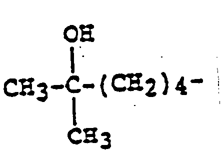 bedeutet
R₂ -CH₃ bedeutet
und
R₃ 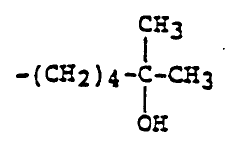 bedeutet.

14. Verwendung gemäß Anspruch 4, worin
R₁ 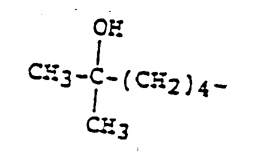 bedeutet,
R₂ -CH₃ bedeutet und
R₃ -CH₂-O-CH₂-CH₃ bedeutet.

15. Verwendung von Pentoxifyllin zur Herstellung eines Medikaments, um die Aktivität von IL-1 oder anderen von Leucozyten abstammenden Cytokinen, die in mit LPS oder Candida albicans stimuliertem konditioniertem Medium mononucleärer Leucozyten enthalten sind, in einem Menschen zu inhibieren, außer für die Behandlung von Osteoporose.

16. Verwendung gemäß Anspruch 15 für die Herstellung eines Medikaments zur Behandlung eines durch ein Virus verursachten krankhaften Zustandes in einem Säugetier.

17. Verwendung gemäß Anspruch 16, wobei das Säugetier ein Mensch ist.

## Patentansprüche (Patentansprüche für folgende(n) Vertragsstaat(en): ES)

1. Verfahren für die Herstellung eines Medikaments für ein Säugetier, um
**a)** Sepsis, septischen Schock, endotoxischen Schock, gramnegative Sepsis, das toxische Schocksyndrom oder Atemschwierigkeiten bei Erwachsenen zu lindern; oder
**b)** Cachexie infolge von Infektion, malignen Tumoren oder AIDS zu lindern; oder
**c)** die Immunantwort zu inhibieren, indem die Aktivität von IL-1 oder anderen von Leukozyten abstammenden Cytokinen, die in mit LPS- oder Candida albicans stimuliertem konditioniertem Medium mononucleärer Leucozyten enthalten sind, auf polymorphonucleare neutrophile Granulozyten gehemmt wird,
das den folgenden Schritt umfaßt:
- Kombination mindestens einer Verbindung der allgemeinen Formel (I) 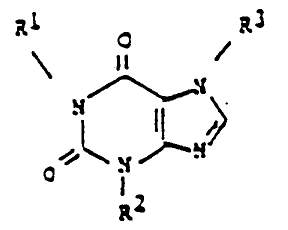
wobei mindestens einer der Reste R₁ und R₃ entweder
**a)** eine verzweigte Hydroxyalkylgruppe der Formel 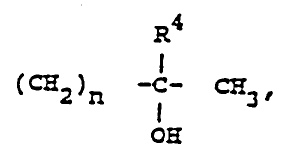 ist, in der R⁴ für eine Alkylgruppe mit 1 bis 3 Kohlenstoffatomen und n für eine ganze Zahl von 2 bis 5 steht, wobei die andere R¹ oder R³-Gruppe, die optional vorhanden sein kann, für ein Wasserstoffatom oder eine aliphatische Kohlenwasserstoffgruppe R⁵ mit bis zu 6 Kohlenstoffatomen steht, deren Kohlenstoffkette durch bis zu 2 Sauerstoffatome unterbrochen sein kann oder mit einer Hydroxy- oder Oxogruppe substituiert sein kann, oder
**b)** eine Oxoalkylgruppe der Formel 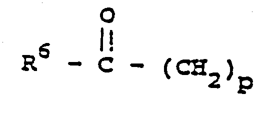 bedeutet, wobei R⁶ C₁-C₆ und p 2 oder 4 bedeutet, wobei die restlichen R¹ oder R³ wie oben definiert sind und R² eine C₁-C₄ Alkylgruppe ist, mit Verdünnungsmitteln, Trägerstoffen und/oder Hilfsstoffen in bekannter Weise.

2. Verfahren gemäß Anspruch 1, wobei das Säugetier ein Mensch ist.

3. Verfahren gemäß Anspruch 1, worin die Verbindung Pentoxifyllin ist.

4. Verfahren gemäß Anspruch 1, worin R¹ eine verzweigte Hydroxyalkylgruppe der Formel 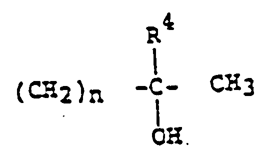 bedeutet, in der R⁴ für eine Alkylgruppe mit 1 bis 3 Kohlenstoffatomen und n für eine ganze Zahl von 2 bis 5 steht.

5. Verfahren gemäß Anspruch 4, worin
R₁ 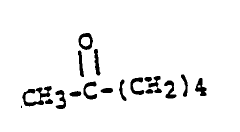
R₂ -CH₃ bedeutet
und
R₃ -CH₂-CH₂-CH₃ bedeutet.

6. Verfahren gemäß Anspruch 4, worin
R₁ 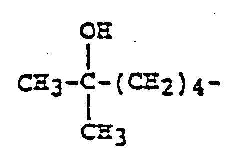
R₂ -CH₃ bedeutet
und
R₃ -CH₂-CH₂-O-CH₃ bedeutet.

7. Verfahren gemäß Anspruch 4, worin
R₁ 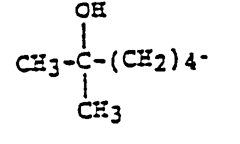 bedeutet,
R₂ -CH₃ bedeutet
und
R₃ -CH₂-O-(CH₂)2-O-CH₃ bedeutet.

8. Verfahren gemäß Anspruch 4, worin
R₁ 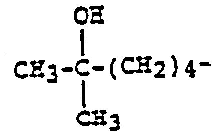 bedeutet,
R₂ -CH₃ bedeutet
und R₃ -H bedeutet.

9. Verfahren gemäß Anspruch 4, worin
R₁ 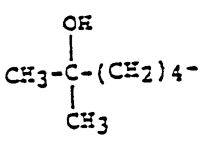 bedeutet,
R₂ -CH₃ bedeutet,
und R₃ -CH₂-CH₂-CH₃ bedeutet

10. Verfahren gemäß Anspruch 4, worin
R₁ 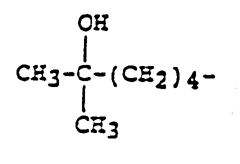 bedeutet,
R₂ -CH₃ bedeutet
und
R₃ 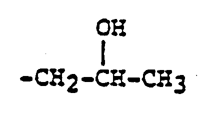 bedeutet.

11. Verfahren gemäß Anspruch 4, worin
R₁ 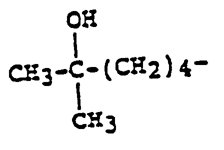 bedeutet,
R₂ -CH₃ bedeutet
und
R₃

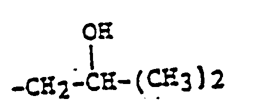

12. Verfahren gemäß Anspruch 4, worin
R₁ 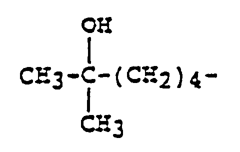 bedeutet,
R₂ -CH₂-CH₃ bedeutet
und
R₃ -CH₂-O-CH₂-CH₃ bedeutet.

13. Verfahren gemäß Anspruch 4, worin
R₁ 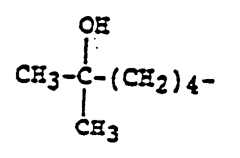 bedeutet,
R₂ -CH₃ bedeutet
und
R₃ 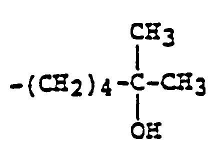 bedeutet.

14. Verfahren gemäß Anspruch 4, worin
R₁ 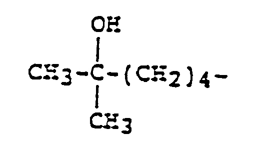 bedeutet,
R₂ -CH₃ bedeutet und
R₃ -CH₂-O-CH₂-CH₃ bedeutet.

15. Verfahren für die Herstellung eines Medikaments, um die Aktivität von IL-1 oder anderen von Leucozyten abstammenden Cytokinen, die in mit LPS oder Candida albicans stimuliertem konditioniertem Medium mononucleärer Leucozyten enthalten sind, in einem Menschen zu inhibieren, außer für die Behandlung von Osteoporose, das die folgenden Schritte enthält:
- Kombination von Pentoxifyllin mit Verdünnungsmitteln, Trägerstoffen und/oder Hilfsstoffen in bekannter Weise.

16. Verfahren gemäß Anspruch 15 für die Herstellung eines Medikaments zur Behandlung eines durch ein Virus verursachten krankhaften Zustandes in einem Säugetier.

17. Verfahren gemäß Anspruch 16, wobei das Säugetier ein Mensch ist.

## Revendications (Revendications pour l'(les) Etat(s) contractant(s) suivant(s): AT, BE, CH, DE, FR, GB, GR, IT, LI, LU, NL, SE)

1. Utilisation d'au moins un composé de formule générale (I) 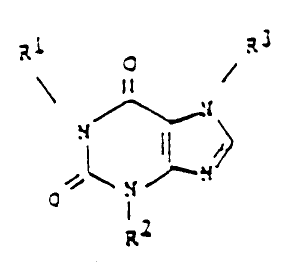 dans laquelle l'un au moins des radicaux R¹ et R³ est
(a) un groupe hydroxyalkyle ramifié de formule 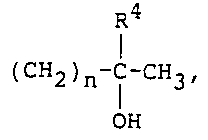 dans laquelle R⁴ désigne un groupe alkyle comportant 1 à 3 atomes de carbone et n désigne un nombre entier de 2 à 5, l'autre radical R¹ ou R³ éventuellement présent représente un atome d'hydrogène ou un groupe hydrocarboné aliphatique R⁵ comportant jusqu'à 6 atomes de carbone, dont la chaîne carbonée peut être interrompue par jusqu'à 2 atomes d'oxygène ou peut être substituée par un groupe hydroxy ou oxo, ou
(b) un groupe oxoalkyle de formule 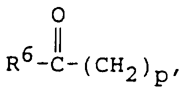 dans laquelle R⁶ est un groupe alkyle en C₁-C₆, et p= 2 ou 4, l'autre radical R¹ ou R³ étant défini comme ci-dessus; et R² représente un groupe alkyle en C₁-C₄;
pour la fabrication d'un médicament pour un mammifère, servant à:
a) atténuer la septicémie, le choc septique, le choc endotoxique, la septicémie à germes Gram négatif, le syndrome du choc toxique ou la détresse respiratoire chez l'adulte; ou
b) atténuer la cachexie secondaire à l'infection, à la malignité ou au SIDA; ou
c) inhiber la réponse immunitaire en inhibant l'activité de IL-1 ou d'autres citokynes dérivées de leucocytes contenus dans un milieu conditionné stimulé par LPS ou C. albicans de leucocytes mononucléaires sur des neutrophiles polynucléaires.

2. Utilisation selon la revendication 1, dans laquelle ledit mammifère est un humain.

3. Utilisation selon la revendication 1, dans laquelle ledit composé est la pentoxifylline.

4. Utilisation selon la revendication 1, dans laquelle R¹ est un groupe hydroxyalkyle ramifié de formule 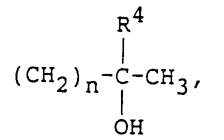 dans laquelle R⁴ représente un groupe alkyle de 1 à 3 atomes de carbone et n représente un nombre entier de 2 à 5.

5. Utilisation selon la revendication 4, dans laquelle:
R¹ est 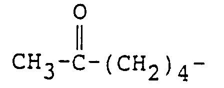
R² est -CH₃
et
R³ est: -CH₂-CH₂-CH₃.

6. Utilisation selon la revendication 4, dans laquelle
R¹ est 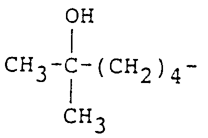
R² est -CH₃
et R³ est -CH₂-CH₂-O-CH₃.

7. Utilisation selon la revendication 4, dans laquelle
R¹ est 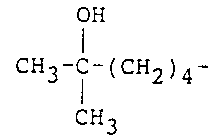
R² est -CH₃
et R³ est
-CH₂-O-(CH₂)₂-O-CH₃

8. Utilisation selon la revendication 4, dans laquelle
R¹ est 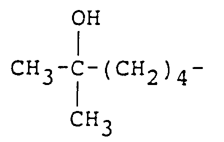
R² est -CH₃
et R³ est -H.

9. Utilisation selon la revendication 4, dans laquelle
R¹ est 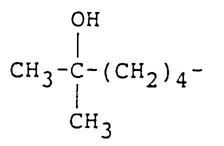
R² est -CH₃
et R₃ est CH₂-CH₂-CH₃.

10. Utilisation selon la revendication 4, dans laquelle
R¹ est 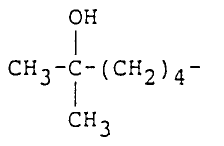
R² est -CH₃
et R³ est

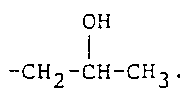

11. Utilisation selon la revendication 4, dans laquelle
R¹ est 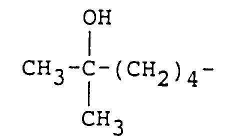
R² est -CH₃
et R³ est

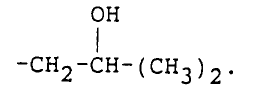

12. Utilisation selon la revendication 4, dans laquelle
R¹ est 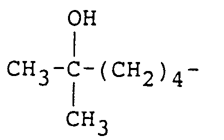
R² est -CH₂-CH₃
et R³ est
-CH₂-O-CH₂-CH₃.

13. Utilisation selon la revendication 4, dans laquelle
R¹ est 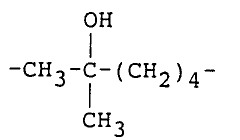
R² est -CH₃
et
R₃ est

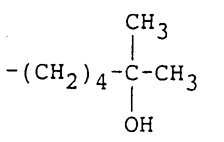

14. Utilisation selon la revendication 4, dans laquelle
R¹ est 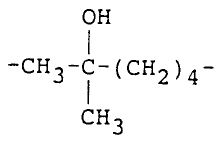
R² est -CH₃
et
R₃ est
-CH₂-O-CH₂-CH₃.

15. Utilisation de la pentoxifylline pour la fabrication d'un médicament servant à inhiber l'activité de IL-1 ou d'autres cytokines dérivées de leucocytes contenus dans un milieu conditionné stimulé par LPS ou C. albicans de leucocytes mononucléaires chez l'homme, à l'exception du traitement de l'ostéoporose.

16. Utilisation selon la revendication 15, pour la fabrication d'un médicament pour traiter un état pathologique provoqué par un virus chez un mammifère.

17. Utilisation selon la revendication 16, dans laquelle ledit mammifère est un humain.

## Revendications (Revendications pour l'(les) Etat(s) contractant(s) suivant(s): ES)

1. Procédé de fabrication d'un médicament pour un mammifère servant à:
a) atténuer la septicémie, le choc septique, le choc endotoxique, la septicémie à germes Gram négatif, le syndrome du choc toxique ou la détresse respiratoire chez l'adulte; ou
b) atténuer la cachexie secondaire à l'infection, à la malignité ou au SIDA; ou
c) inhiber la réponse immunitaire en inhibant l'activité de IL-1 ou d'autres citokynes dérivées de leucocytes contenus dans un milieu conditionné stimulé par LPS ou C. albicans de leucocytes mononucléaires sur des neutrophiles polynucléaires,
comprenant l'étape qui consiste à combiner au moins un composé de formule générale (I) 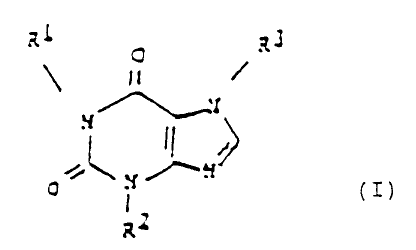 dans laquelle l'un au moins des radicaux R¹ et R³ est
(a) un groupe hydroxyalkyle ramifié de formule 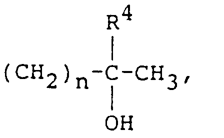 dans laquelle R⁴ désigne un groupe alkyle comportant 1 à 3 atomes de carbone et n désigne un nombre entier de 2 à 5, l'autre radical R¹ ou R³ éventuellement présent représente un atome d'hydrogène ou un groupe hydrocarboné aliphatique R⁵ comportant jusqu'à 6 atomes de carbone, dont la chaîne carbonée peut être interrompue par jusqu'à 2 atomes d'oxygène ou peut être substituée par un groupe hydroxy ou oxo, ou
(b) un groupe oxoalkyle de formule 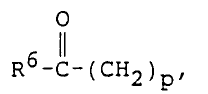 dans laquelle R⁶ est un groupe alkyle en C₁-C₆, et p= 2 ou 4, l'autre radical R¹ ou R³ étant défini comme ci-dessus; et R² représente un groupe alkyle en C₁-C₄,
avec des diluants, des véhicules et/ou des excipients, d'une manière connue en soi.

2. Procédé selon la revendication 1, dans lequel ledit mammifère est un humain.

3. Procédé selon la revendication 1, dans lequel ledit composé est la pentoxifylline.

4. Procédé selon la revendication 1, dans lequel R¹ est un groupe hydroxyalkyle ramifié de formule 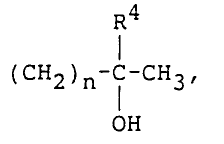 dans laquelle R⁴ représente un groupe alkyle de 1 à 3 atomes de carbone et n représente un nombre entier de 2 à 5.

5. Procédé selon la revendication 4, dans lequel:
R¹ est 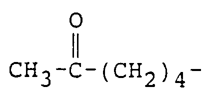
R² est -CH₃
et
R³ est: -CH₂-CH₂-CH₃.

6. Procédé selon la revendication 4, dans lequel
R¹ est 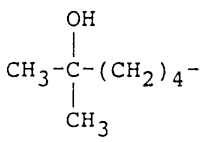
R² est -CH₃
et R³ est -CH₂-CH₂-O-CH₃.

7. Procédé selon la revendication 4, dans lequel
R¹ est 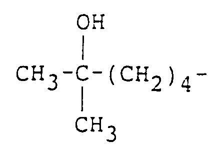
R² est -CH₃
et R³ est -CH₂-O-(CH₂)₂-O-CH₃.

8. Procédé selon la revendication 4, dans lequel
R¹ est 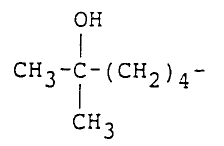
R² est -CH₃
et R³ est -H.

9. Procédé selon la revendication 4, dans lequel
R¹ est 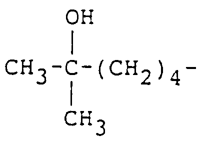
R² est -CH₃
et R₃ est CH₂-CH₂-CH₃.

10. Procédé selon la revendication 4, dans lequel
R¹ est 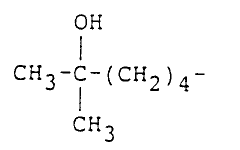
R² est -CH₃
et R³ est

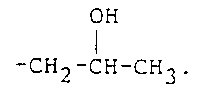

11. Procédé selon la revendication 4, dans lequel
R¹ est 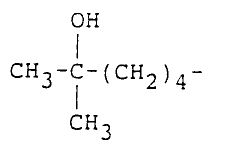
R² est -CH₃
et R³ est

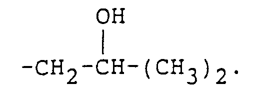

12. Procédé selon la revendication 4, dans lequel
R¹ est 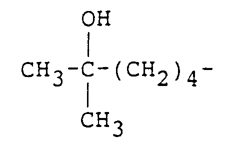
R² est -CH₂-CH₃
et R³ est
CH₂-O-CH₂-CH₃.

13. Procédé selon la revendication 4, dans lequel
R¹ est 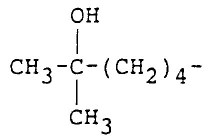
R² est -CH₃
et R³ est

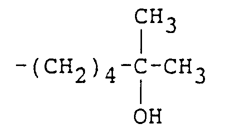

14. Procédé selon la revendication 4, dans lequel
R¹ est 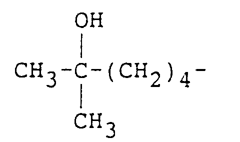
R² est -CH₃
et R³ est -CH₂-O-CH₂-CH₃.

15. Procédé de fabrication d'un médicament servant à inhiber l'activité de IL-1 ou d'autres cytokines dérivées de leucocytes contenus dans un milieu conditionné stimulé par LPS ou C. albicans de leucocytes mononucléaires chez l'homme, à l'exception du traitement de l'ostéoporose, comprenant les étapes consistant à combiner la pentoxifylline avec des diluants, des véhicules et/ou des excipients d'une manière connue en soi.

16. Procédé selon la revendication 15, dans lequel on fabrique un médicament pour traiter un état pathologique provoqué par un virus chez un mammifère.

17. Procédé selon la revendication 16, dans lequel ledit mammifère est un humain.
